# EUROPÄISCHE PATENTSCHRIFT

(11) **EP 2 856 020 B1**
(45) Veröffentlichungstag und Bekanntmachung des Hinweises auf die Patenterteilung: **06.07.2016**
(21) Anmeldenummer: 13727118.5
(22) Anmeldetag: 31.05.2013
(51) Int. Cl.: A61L 2/08

(54) **VORRICHTUNG ZUM VERWENDEN IN EINEM MEDIZINISCHEN BEHANDLUNGSRAUM**
DEVICE FOR USE IN A MEDICAL TREATMENT ROOM
DISPOSITIF DESTINÉ À ÊTRE UTILISÉ DANS UN LOCAL DE TRAITEMENT MÉDICAL

(30) Priorität: 31.05.2012 DE 102012010676
(43) Veröffentlichungstag der Anmeldung: 08.04.2015
(73) Patentinhaber: Drägerwerk AG & Co. KGaA, 23558 Lübeck (DE)
(72) Erfinder: KRETSCHMANN, Hanno, 22147 Hamburg (DE)
(74) Vertreter: Guthöhrlein, Gerhard
(86) Internationale Anmeldenummer: PCT/EP2013/061254
(87) Internationale Veröffentlichungsnummer: WO 2013/178781

(56) Entgegenhaltungen:
- WO-A2-99/50593
- KR-A- 20110 089 385
- US-A1- 2009 227 847

## Beschreibung

Die Erfindung ist in den Ansprüchen definiert und betrifft eine Vorrichtung zum Verwenden in einem medizinischen Behandlungsraum, umfassend wenigstens eine Bedieneinheit.

In medizinischen Behandlungsräumen werden Vorrichtungen verwendet, die beispielsweise dazu dienen, lebenserhaltende Funktionen von Patienten zu steuern, zu überwachen und/oder einen Arzt bei der Durchführung von Behandlungen zu unterstützen. Damit die Vorrichtungen durch einen Menschen bedient werden können, weisen sie Bedieneinheiten auf. Bei einer Bedieneinheit handelt es sich beispielsweise um einen Handgriff, mittels dessen die Vorrichtung in eine gewünschte Position bzw. an einen gewünschten Ort gebracht werden kann. Weiterhin kann eine Bedieneinheit ein Schalter oder ein Schaltfeld mit Sensoren sein, deren Betätigung zur Aktivierung oder Deaktivierung von Betriebszuständen der Vorrichtung führt.

Trotz vorheriger Sterilisierung oder Desinfektion der Oberflächen der Bedieneinheiten kann es zu einer Kontamination mit insbesondere pathogenen Bakterien kommen. Dies kann dadurch geschehen, dass an der Hand eines die Vorrichtung bedienenden Menschen befindliche Bakterien beim Bedienen der Vorrichtung auf die Oberfläche der Bedieneinheit gelangen. Dies kann wiederum dazu führen, dass während der Behandlung eines Patienten Bakterien an die Hände weiterer, die Vorrichtung bedienender Personen gelangen und somit ein erhöhtes Risiko dafür besteht, dass ein behandelter Patient mit pathogenen Bakterien in Kontakt kommt. Besonderes hoch ist dieses Risiko bei Vorrichtungen, die in einer sterilen Umgebung wie beispielsweise einem Operationssaal oder einem Zimmer mit einem intensivmedizinisch versorgten Patienten verwendet werden.

Dokument WO 98/50593 offenbart eine lichtquelle mit Boedieneinheit wie sie zum Stand der Tecknik gehört.

Der Erfindung liegt die Aufgabe zugrunde, eine vorteilhafte Vorrichtung zum Verwenden in einem medizinischen Behandlungsraum, umfassend eine Bedieneinheit anzugeben, bei der eine Kontamination der Bedieneinheit mit pathogenen Bakterien behindert wird.

Diese Aufgabe wird mit der Vorrichtung gemäß Anspruch 1 gelöst.

Vorteilhafte Ausgestaltungen und Weiterbildungen der Erfindung sind Gegenstand der abhängigen Ansprüche.

Die erfindungsgemäße Vorrichtung zum Verwenden in einem medizinischen Behandlungsraum umfasst wenigstens eine als Lichtleiter ausgebildete Bedieneinheit und wenigstens eine Lichtquelle, die Licht mit einer Wellenlänge zwischen 380 Nanometern und 420 Nanometern, insbesondere mit 405 Nanometern, emittiert und derart angeordnet ist, dass emittiertes Licht in den Lichtleiter eingekoppelt wird, wobei der Lichtleiter derart ausgebildet ist, dass aus dem Lichtleiter wieder austretendes Licht die Oberfläche der Bedieneinheit zumindest teilweise durchleuchtet.

Der Erfinder hat erkannt, dass die in der WO 2007/012875 A1 beschriebene Auswirkung einer Bestrahlung von pathogenen Bakterien mit sichtbarem Licht im Bereich von 400 - 500 nm dazu genutzt werden kann, durch Bestrahlen oder Durchstrahlen einer Bedieneinheit einer Vorrichtung mit Licht in diesem Wellenlängenbereich die Kontamination der Bedieneinheit mit pathogenen Bakterien zu verringern oder im Idealfall sogar zu verhindern. Die erfindungsgemäße Vorrichtung lässt sich deshalb besonders vorteilhaft unter sterilen Bedingungen verwenden, wie sie in Operationssälen oder Zimmern mit intensivmedizinisch versorgten Patienten herrschen müssen.

Dadurch, dass die Lichtquelle Bestandteil der erfindungsgemäßen Vorrichtung ist, d.h. durch Anordnen der Lichtquelle in bzw. an der Vorrichtung kann die Lichtquelle derart benachbart zur Oberfläche der Bedieneinheit, d.h. mit wenigen Zentimetern Abstand oder beispielsweise mittels Lichtleitern unmittelbar hinter der der Oberfläche angeordnet werden, dass als Lichtquelle beispielsweise eine oder auch mehrere LED mit einer Gesamtleistung von wenigen Watt verwendet werden kann, um auf einer Bedieneinheit mit einer Oberfläche von beispielsweise 50 cm² eine Bestrahlungsstärke zu erreichen, die eine signifikante Reduzierung pathogener Bakterien in einigen Sekunden bis Minuten bewirkt. Beispielsweise liegt die Gesamtleistung der Lichtquelle im Bereich von 1 Watt bis 10 Watt und die Strahlungsintensität an der Oberfläche der Bedieneinheit zwischen 0,1 bis 10 Watt pro Quadratzentimeter. Für eine Reduzierung der pathogenen Bakterien um fünf Größenordnungen wird eine Dosis von etwa 50 Joule pro Quadratzentimeter benötigt. Diese Dosis wird bei einer Oberfläche von beispielsweise 50 cm² und einer die Oberfläche be- oder durchstrahlenden Leistung von 10 Watt in 250 Sekunden erreicht.

Wird die Oberfläche der Bedieneinheit der erfindungsgemäßen Vorrichtung zunächst sterilisiert, kann sie dadurch im Wesentlichen steril gehalten werden, dass die Lichtquelle nach einem Berühren durch eine Person solange angeschaltet bleibt, bis eine Reduzierung der Batterien um beispielsweise fünf Größenordnungen bewirkt worden ist. Von Vorteil ist, wenn die Lichtquelle zusätzlich während des Berührens angeschaltet ist.

Im Gegensatz dazu ist für eine Bestrahlung einer Oberfläche mit einer separaten und weit von der Oberfläche entfernt angeordneten Lichtquelle, wie sie beispielsweise in der WO 2009/056838 A1 beschrieben ist, eine erheblich höhere Leistung der Lichtquelle erforderlich, um bei einem Abstand zur bestrahlten Fläche von 1 bis 2 Metern noch eine signifikante Reduzierung pathogener Bakterien erzielen zu können. Darüber hinaus benötigt die dort beschriebene Lichtquelle mehr als 8 Stunden, um eine signifikante Reduzierung pathogener Bakterien zu erreichen. Für eine kürzere Zeit wäre eine noch höhere Leistung der Lichtquelle erforderlich.

Ein Lichtleiter ist dabei jeder Körper, in dem eingekoppeltes Licht mehrfach zwischen den Körper begrenzenden Wänden hin und her reflektiert wird. Dabei können die Wände des Lichtleiters so ausgestaltet sein, dass das Licht bei jeder Reflexion nur teilweise reflektiert wird, d.h. ein Teil des Lichts gelangt durch die Wand. Im einfachsten Fall ist ein Lichtleiter ein Körper, beispielsweise aus Glas, aus Acrylglas oder aus einem anderen Kunststoff, aus dessen gesamter Oberfläche eingekoppeltes Licht heraustritt.

Mit Vorteil sind im Lichtleiter und/oder an dessen Oberflächen lichtlenkende Strukturen derart ausgebildet und angeordnet, dass eingekoppeltes Licht in Richtung der Oberfläche der Bedieneinheit gelenkt wird.

Die Auskopplung kann durch im Lichtleitermaterial verteilte streuende Strukturen, durch gezielte feine Oberflächenstrukturen oder durch feine, aufgedruckte Muster realisiert werden. Dabei bewirken diese lichtlenkenden Strukturen, dass eingekoppeltes Licht durch Reflexion, Diffraktion, Refraktion oder Streuung bevorzugt in Richtung der beim Bedienen der Bedieneinheit berührte Oberfläche gelenkt und dann zumindest teilweise über die beim Bedienen berührte Oberfläche ausgekoppelt wird. Beispielsweise sind lichtlenkende Strukturen im Millimeter- oder Mikrometerbereich ausgebildeten Fresnel-Strukturen. Die Abmessungen von diffraktiven Strukturen können insbesondere auch im Nanometerbereich liegen. Die inhomogene Verteilung der auskoppelnden Strukturen bewirkt, dass eine gleichmäßige, insbesondere homogene Durchleuchtung der Fläche auch mit nur einer an einer Seite eingekoppelten Lichtquelle erreicht wird.

Durch die Ausgestaltung der Bedieneinheit als Lichtleiter genügt es, das Licht der Lichtquelle an einer Seite in die Bedieneinheit einzukoppeln, um die ganze Oberfläche der Bedieneinheit zu durchleuchten. Dadurch, dass das Licht innerhalb der Bedieneinheit nicht vollständig reflektiert wird, sondern teilweise zumindest über die beim Bedienen berührte Oberfläche austritt, wird diese durchleuchtet und darauf befindliche pathogene Bakterien abgetötet. Dieser Effekt kann dadurch begünstigt werden, dass der Lichtleiter so ausgebildet ist, dass Licht ganz überwiegend aus der beim Bedienen berührten Oberfläche der Bedieneinheit austritt und nur in einem geringen Maße oder gar nicht aus anderen Oberflächen der Bedieneinheit heraus austreten kann. Dies kann beispielsweise durch eine geeignete Beschichtung der anderen Oberflächen erreicht werden.

Ein im Wesentlichen homogenes Durchleuchten oder Beleuchten der Oberfläche der Bedieneinheit, d.h. eine homogene Intensitätsverteilung auf der Oberfläche, kann auch durch das Aufbringen zusätzlicher Strukturen, beispielsweise in Form von Lacken, Schichten oder Partikeln, auf die Oberfläche bewirkt werden. Die Ausdehnung solcher Strukturen kann im Nano-, Mikro- oder auch Millimeterbereich liegen.

In einer Ausgestaltung der Erfindung ist die Bedieneinheit als Handgriff ausgebildet.

Alternativ oder zusätzlich zur Ausbildung als Handgriff umfasst die Bedieneinheit vorteilhafterweise wenigstens eine mit einem Tastsensor in Wirkverbindung stehende Tastfläche, die derart ausgebildet und/oder angeordnet ist, dass die Tastfläche bei aktivierter Lichtquelle von dem emittierten Licht durchleuchtet wird.

Auf diese Weise kann gezielt auf den für eine Berührung durch eine die Vorrichtung bedienende Person vorgesehenen Tastflächen durch die Bestrahlung mit Licht die Anzahl pathogener Bakterien durch Abtöten verringert oder im Idealfall auf Null reduziert werden.

Mit Vorteil ist die Bedieneinheit aus transparentem Kunststoff oder aus Glas gefertigt.

Vorteilhafterweise ist die Bedieneinheit als Touchscreen ausgebildet.

In einer bevorzugten Ausgestaltung der Erfindung ist die Vorrichtung eine Operationsleuchte.

Operationsleuchten können auch durch den die Operation durchführenden Arzt bedient werden. Dabei berührt er Bedieneinheiten, beispielsweise Handgriffe und/oder Tastelemente, insbesondere Touchscreens, die an der Operationsleuchte, beispielsweise am Rand außerhalb des Lichtkegels des Operationslichtes, angebracht sind. Die Tastelemente können selbstverständlich auch an der Ober- oder Unterseite der Operationsleuchte, insbesondere in einem Randbereich integriert ausgebildet sein.

Wenn der Operateur während der Operation nicht nach jeder Berührung von Bedieneinheiten der Operationsleuchte die Hände desinfiziert oder sterilisiert und/oder neue Handschuhe anzieht, kann nicht ausgeschlossen werden, dass durch das Berühren der Oberflächen dieser Bedieneinheiten pathogene Bakterien an die Hände bzw. Handschuhe des Operateurs gelangen. Alternativ kann der Operateur Bedieneinheiten der Operationsleuchte auch durch bei der Operation anwesendes Personal berühren lassen. Hierzu muss er allerdings während der Operation immer wieder Anweisungen geben. Dies ist aufwendig und kann unter Umständen auch die Konzentration des Operateurs beeinträchtigen. Insbesondere für die Oberflächen von Bedieneinheiten einer Operationsleuchte ist daher eine möglichst wirksame und kontinuierliche Abtötung pathogener Bakterien erforderlich und kann durch die erfindungsgemäße Ausgestaltung der Operationsleuchte erreicht werden.

In einer alternativen Ausgestaltung ist die Vorrichtung eine Versorgungseinheit zum Anschließen eines Medizinprodukts. Versorgungseinheiten sind in Operationssälen oder Behandlungszimmern beispielsweise über ein bewegliches Armsystem an der Decke befestigt und dienen dazu, Medizinprodukte wie beispielsweise Anästhesiegeräte, Beatmungsgeräte oder Wärmetherapiegeräte beispielsweise an eine Gasversorgung und/oder an elektrische Energie anzuschließen. Versorgungseinheiten weisen ebenfalls Bedieneinheiten auf, die mittels der erfindungsgemäßen Anordnung einer Lichtquelle mit Licht im Wellenlängenbereich zwischen 380 nm und 420 nm wirksam vor einer übermäßigen Kontamination mit pathogenen Bakterien geschützt werden können.

Die Erfindung wird im Folgenden anhand von in den Figuren dargestellten Ausführungsbeispielen näher erläutert. Es zeigen:
- Fig. 1: eine erfindungsgemäße Vorrichtung in Form einer Operationsleuchte,
- Fig. 2: einen mit Licht durchleuchteten Handgriff zur Positionierung und Steuerung der Operationsleuchte gemäß Fig. 1 und
- Fig. 3: eine Versorgungseinheit zum Anschließen eines Medizinproduktes.

Gleiche Bezugszeichen in den Figuren bezeichnen gleiche Gegenstände.

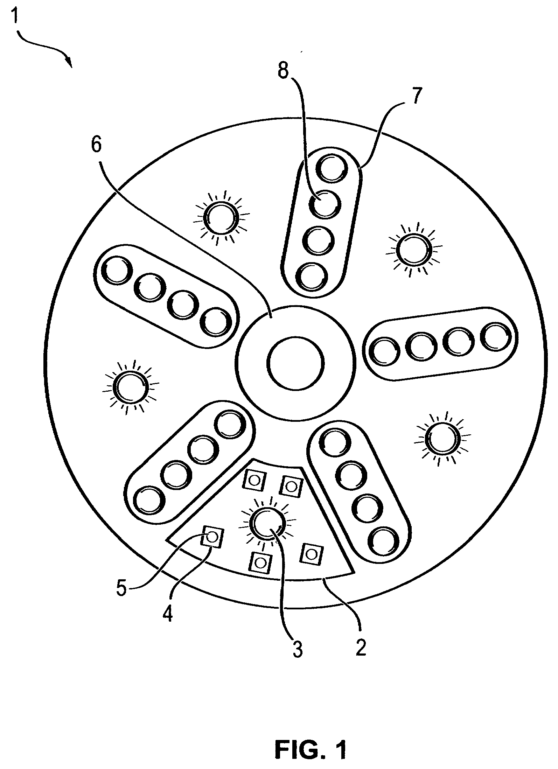

Fig. 1 zeigt schematisch eine Operationsleuchte 1. Gezeigt ist ein Blick senkrecht auf den das Operationslicht emittierenden Teil der Operationsleuchte 1. Der Strahlengang des Lichts ist dabei aus der Zeichenebene heraus gerichtet. Zur Erzeugung des Operationslichtes sind beispielsweise sechs Module 7 mit jeweils beispielsweise vier Leuchtmitteln 8 beispielsweise sternförmig um das Zentrum der Operationsleuchte 1 herum angeordnet. Die Leuchtmittel 8 sind beispielsweise weiße LEDs, mittels derer in bekannter Weise ein Operationsfeld ausgeleuchtet werden kann. Alternativ können die Leuchtmittel 8 auch aus verschieden farbigen LEDs, Gasentladungslampen, Glühlampen und/oder Halogenlampen gebildet werden. Anstatt mittels Modulen können die Leuchtmittel 8 selbstverständlich auch einzeln in der Operationsleuchte 1 angeordnet sein. Der Übersichtlichkeit halber sind nur ein Modul 7 und ein Leuchtmittel 8 mit Bezugsziffern versehen.

Weiterhin weist die Operationsleuchte 1 sechs Lichtquellen 3 auf, von denen wiederum nur eine mit einem Bezugszeichen versehen wurde. Die Lichtquellen 3 sind beispielsweise LEDs oder Xenonlampen, die Licht im Bereich von 380 bis 420 nm emittieren. Bevorzugt wird Licht jedoch mit 405 nm emittiert. Dazu können die LEDs so ausgebildet sein, dass das emittierte Spektrum schmalbandig (beispielsweise 15nm bis 20nm FWHM) ist und sein Maximum bei 405 nm hat. Die Lichtquellen 3 sind dabei so ausgerichtet, dass bei einer Positionierung der Operationsleuchte 1 über einem Wundraum das von den Lichtquellen 3 emittierte Licht den Wundraum und daneben liegende Bereiche bestrahlt, sodass pathogene Bakterien im Wundraum sowie dem umgebenden Bereich abgetötet werden. Hierzu sind die Lichtquellen 3 bei einem Abstand der Operationsleuchte 1 vom Wundraum von ca. 100 cm und einer Wundfläche von ca. 100 cm² - 300 cm² so ausgelegt, dass auf der Wundfläche eine Strahlungsintensität von 1 - 10 W/m² vorliegt. Zusätzlich kann eine nicht dargestellte Optik vorgesehen sein, mit der das von den Lichtquellen 3 emittierte Licht auf den Wundraum und den umgebenden Bereich fokussiert werden kann. Diese Optik sowie die Leistung der Lichtquellen 3 und/oder der Leuchtmittel 8 kann durch das Betätigen von hinter Tastfeldern 4 hinterlegten Tastsensoren 5 gesteuert werden. Die Tastfelder 4 und Tastsensoren 5 sind an einer als Touchscreen ausgeführten Bedieneinheit 2 ausgebildet. Die Bedieneinheit 2 ist beispielsweise aus Glas, kann aber auch aus transparentem Kunststoff gefertigt sein. Vorzugsweise ist die Bedieneinheit 2 Bestandteil einer die Lichtquellen 3 und die Leuchtmittel 8 abdeckenden, in Fig. 1 nicht dargestellten Glasplatte oder in diese Glasplatte integriert.

Eine der Lichtquellen 3 ist hinter der Bedieneinheit 2 angeordnet, sodass von der Lichtquelle 3 emittiertes Licht die Bedieneinheit 2 durchleuchtet und somit auch ein Abtöten pathogener Bakterien bewirkt, die sich auf der Oberfläche der Bedieneinheit 2 befinden können. Die Oberfläche der Bedieneinheit ist dabei beispielsweise durch eine geeignete Beschichtung so strukturiert, dass die Intensitätsverteilung des Lichtes auf der Oberfläche im Wesentlichen homogen ist.

Die Operationsleuchte 1 weist weiterhin eine Bedieneinheit 6 auf, die als Handgriff ausgebildet ist, der beispielsweise zentral im Bereich der Lichtaustrittsfläche der Operationsleuchte 1 angeordnet ist und zur Positionierung der Operationsleuchte 1 relativ zu einem zu behandelnden Patienten dient.

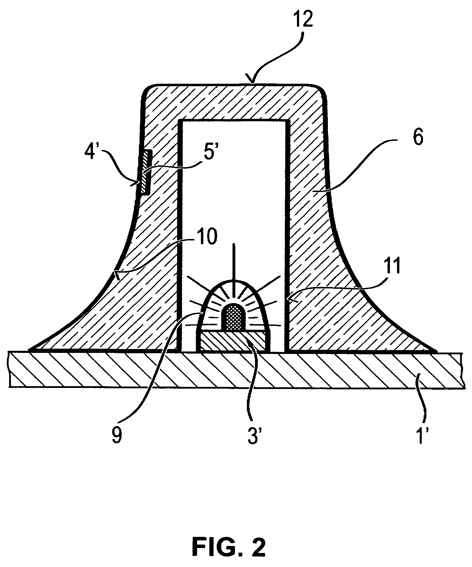

Fig. 2 zeigt schematisch einen Schnitt durch den Handgriff 6 gemäß Fig. 1. Die Schnittebene geht dabei mittig durch den Handgriff 6 und liegt senkrecht zur Zeichenebene der Figur 1. Der Handgriff 6 ist an einer Fläche 1' im Bereich der Lichtaustrittsfläche der Operationsleuchte 1 befestigt. Die Befestigung kann durch Schrauben, Kleben oder andere geeignete Methoden erfolgen. Der Handgriff 6 weist innen einen Hohlraum auf, in den eine weitere Lichtquelle 3' hineinragt, die aus Gründen der Übersichtlichkeit in Fig. 1 nicht dargestellt wurde. Die weitere Lichtquelle 3' emittiert ebenso wie die Lichtquellen 3 Licht mit einer Wellenlänge zwischen 380 nm und 420 nm, insbesondere mit 405 nm. Die weitere Lichtquelle 3' kann insbesondere hinsichtlich der Strahlungsquelle und des emittierten Spektrums baugleich zu den Lichtquellen 3 ausgeführt sein. Oberhalb der weiteren Lichtquelle 3' ist eine Optik 9 angeordnet, mittels derer das Licht gleichmäßig in den Hohlraum abgestrahlt wird, sodass Licht innerhalb des als Lichtleiter ausgebildeten, beispielsweise aus transparentem Kunststoff bestehenden Handgriffs 6 zwischen Wänden 10 und 11 hin und her reflektiert wird und über die Wand 10 und eine Wand 12 aus dem Handgriff 6 wieder austritt. Auf diese Weise wird die Oberfläche des Handgriffs 6 durch Licht insbesondere der Wellenlänge 405 nm durchleuchtet, und auf der Oberfläche des Handgriffs 6 befindliche pathogene Bakterien werden abgetötet.

Die Wand 11 weist vorzugsweise eine in der Figur nicht dargestellte Oberflächenstruktur beispielsweise eine Anordnung von im Millimeter- oder Mikrometerbereich ausgebildeten Fresnel-Strukturen auf, die so ausgebildet und angeordnet sind, dass in den Handgriff eingekoppeltes Licht, das nach einer Reflexion an der Wand 10 auf die Wand 11 trifft, gezielt in Richtung der Wand 10 zurückreflektiert wird und dort zumindest teilweise wieder austritt. Dadurch wird erreicht, dass ganz überwiegend die beim Bedienen berührte Oberfläche des Handgriffs durchleuchtet und nur ein geringer, im Idealfall gegen Null gehender Teil des eingekoppelten Lichts wieder in den Hohlraum eintritt.

Der Handgriff 6 weist weiterhin eine Tastfläche 4' und einen Tastsensor 5' auf, über den beispielsweise die Fokussierung und/oder die Leistung der Leuchtmittel 8, der Lichtquellen 3 und/oder der weiteren Lichtquelle 3' steuerbar ist.

Alternativ oder zusätzlich kann die Operationsleute wenigstens einen als Lichtleiter aus Glas oder Kunststoff ausgeführten Touchscreen aufweisen, der beispielsweise in die Ober- oder Unterseite in einem Randbereich der Operationsleuchte integriert ist. In den Lichtleiter wird Licht einer im Wellenlängenbereich von 380 nm bis 420 nm, insbesondere 405 nm, emittierenden Lichtquelle, beispielsweise einer Lichtquelle 3, eingekoppelt. Der Lichtleiter ist dabei, beispielsweise durch eine entsprechende Anordnung von im Millimeter- oder Mikrometerbereich ausgebildeten Fresnel-Strukturen oder durch in den Lichtleiter eingebrachte streuende, reflektierende, refraktive oder diffraktive Strukturen, so ausgeführt, dass das Licht überwiegend zu der Seite austritt auf der die Bedienung erfolgt. Die Abmessungen von diffraktiven Strukturen können insbesondere auch im Nanometerbereich liegen. Darüber hinaus ist die Oberfläche so ausgestaltet, dass die Intensitätsverteilung auf der Oberfläche im Wesentlichen homogen ist. Selbstverständlich kann ein entsprechender Touchscreen auch als gesondertes Bauteil am Rand der Operationsleuchte befestigt sein. Das eingekoppelte Licht kann dann entweder aus einer in das Bauteil integrierten Lichtquelle oder durch einkoppeln von Licht einer der Lichtquellen 3 der Operationsleute erfolgen.

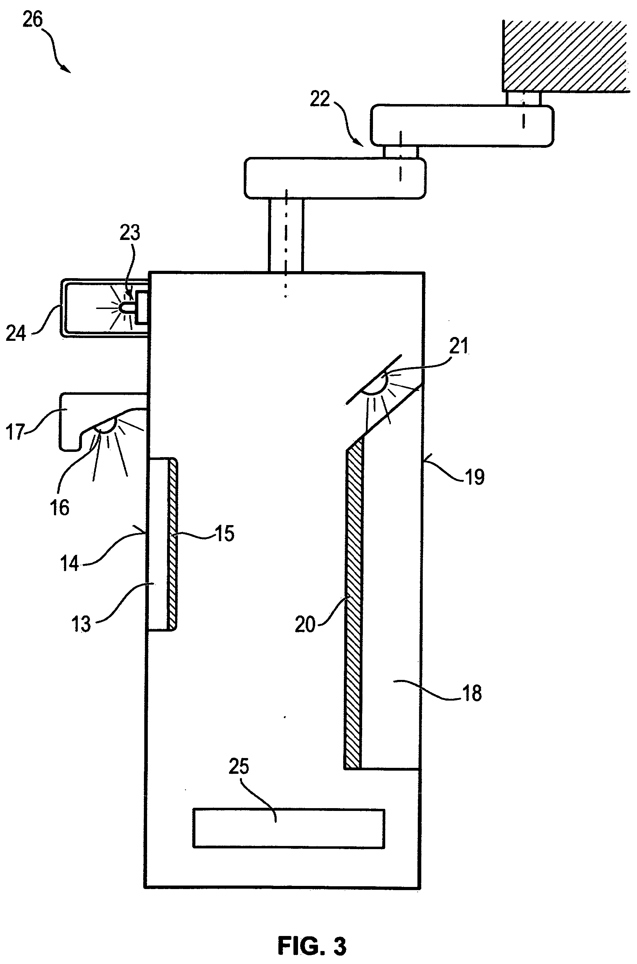

Fig. 3 zeigt schematisch eine Versorgungseinheit zum Anschließen eines Medizinproduktes. Die Versorgungseinheit 26 ist beispielsweise über einen Gelenkarm 22 an einer Decke befestigbar. Über ein Anschlussmodul 25 kann zumindest ein Medizinprodukt, beispielsweise ein Beatmungsgerät für die Intensivmedizin an der Versorgungseinheit 26 angeschlossen werden. Die Versorgungseinheit stellt beispielsweise für ein über das Anschlussmodul angeschlossenes Medizinprodukt einen Anschluss an eine Gasversorgung und an elektrische Energie bereit. Die Versorgungseinheit 26 hat als Bedieneinheit einen Handgriff 24 aus transparentem Kunststoff, der in seinem Inneren hohl ausgeführt ist und von einer Lichtquelle 23 mit Licht der Wellenlänge 405 nm durchleuchtet wird. Weiterhin ist beispielsweise zur Steuerung eines an das Anschlussmodul 25 angeschlossenen Beatmungsgerätes als Bedieneinheit ein Touchscreen 13 vorgesehen, der auf seiner Rückseite mit einer Bedienelektrode 15 beschichtet ist. Um eine Tastfläche 14 des Touchscreens 13 ebenfalls möglichst frei von pathogenen Bakterien zu halten, ist mittels eines Halters 17 eine weitere Lichtquelle 16 an der Versorgungseinheit 26 angeordnet, die Licht insbesondere mit 405 nm emittiert, und auf die Oberfläche 14 gerichtet ist. Die Oberfläche 14 wird somit von Licht der Wellenlänge 405 nm beleuchtet, und darauf befindliche pathogene Bakterien werden abgetötet.

Weiterhin weist die Versorgungseinheit 26 einen Touchscreen 18 aus Glas auf, der beispielsweise dazu dient, in dem Gelenkarm 22 befindliche Motoren anzusteuern und somit die Versorgungseinheit 26 zu positionieren. Der weitere Touchscreen 18 weist auf seiner Rückseite eine Bedienelektrode 20 auf. Der Touchscreen 18 ist als Lichtleiter aus Glas ausgeführt, und in ihn wird Licht der Wellenlänge 405 nm eingekoppelt, das von einer weiteren Lichtquelle 21 emittiert wird. Dieses Licht wird im Touchscreen 18 zwischen einer Oberfläche 19 und der Bedienelektrode 20 hin und her reflektiert und tritt zumindest teilweise über die Oberfläche 19 aus. Durch das über die Oberfläche 19 austretende Licht werden auf der Oberfläche 19 befindliche pathogene Bakterien abgetötet.

Vorzugsweise ist auf der Bedienelektrode 20 eine in der Figur nicht dargestellte Oberflächenstruktur, beispielsweise eine Anordnung von im Millimeter- oder Mikrometerbereich ausgebildeten Fresnel-Strukturen, ausgebildet, durch die eingekoppeltes Licht gezielt in Richtung der Oberfläche 19 reflektiert wird, sodass eingekoppeltes Licht überwiegend durch die Oberfläche 19 austritt. Besonders bevorzugt weisen alle weiteren den Touchscreen begrenzenden Seiten mit Ausnahme der Oberfläche 19 eine reflektierende Beschichtung auf. Alternativ oder zusätzlich sind in den Lichtleiter streuende, refraktierende oder diffraktierende Strukturen eingebrachte und so ausgebildet und angeordnet, dass eingekoppeltes Licht überwiegend in Richtung der Oberfläche 19 gelenkt wird, sodass es überwiegend dort austritt. Um ein Auskoppeln des auf die Oberfläche 19 treffenden Lichtes zu erleichtern, kann die Oberfläche 19 eine refraktive Beschichtung aufweisen.

Die Lichtquellen 16, 21 und 23 sind beispielsweise insbesondere hinsichtlich ihrer Strahlungsquellen und ihrer Spektren baugleich zu den Lichtquellen 3 ausgeführt.

Die Versorgungseinheit 26 ist zusätzlich auch als Medizinprodukt ausgestaltet und weist beispielsweise einen in Figur 3 aus Gründen der Übersichtlichkeit nicht dargestellten Defibrillator aus. Weitere Medizinprodukte können selbstverständlich ebenfalls unmittelbar in der Versorgungseinheit 26 integriert sein.

Selbstverständlich können mit Vorteil auch Bedieneinheiten, d. h. Handgriffe und Touchscreens sowie andere, Tastsensoren aufweisende Bedienoberflächen, von Medizinprodukten, wie beispielsweise Anästhesiegeräten, Beatmungsgeräten und Inkubatoren, von Lichtquellen beleuchtet und/oder durchleuchtet werden, die Licht mit einer zentralen Wellenlänge zwischen 380 nm und 420 nm, insbesondere mit 405 nm emittieren.

Die Ansführungsbeispiele, die nicht unter den Schutzumfang der beigefügten Ansprüche fallen, dinen lediglich zu illustrativen Zwecken und sind nicht Giegendant der gegenwortigen Erfindung.

Die Erfindung wird durch die folgenden Ansprüche definiert.

### BEZUGSZEICHENLISTE

- 1: Operationsleuchte
- 1': Fläche
- 2: Bedieneinheit
- 3, 3': Lichtquelle
- 4, 4': Tastfläche
- 5, 5': Tastsensor
- 6: Hangriff
- 7: Modul
- 8: Leuchtmittel
- 9: Optik
- 10: Wand
- 11: Wand
- 12: Wand
- 13: Touchscreen
- 14: Tastfläche
- 15: Bedienelektrode
- 16: Lichtquelle
- 17: Halter
- 18: Touchscreen
- 19: Tastfläche
- 20: Bedienelektrode
- 21: Lichtquelle
- 22: Gelenkarm
- 23: Lichtquelle
- 24: Handgriff
- 25: Anschlussmodul
- 26: Versorgungseinheit

## Patentansprüche

1. Vorrichtung (1; 26) zum Verwenden in einem medizinischen Behandlungsraum, umfassend wenigstens eine als Lichtleiter ausgebildete Bedieneinheit (2, 6; 18) und wenigstens eine Lichtquelle (3, 3'; 21), **dadurch gekennzeichnet, daß** die lichtquelle Licht mit einer Wellenlänge zwischen 380 Nanometern und 420 Nanometern, insbesondere mit 405 Nanometern, emittiert und derart angeordnet ist, dass emittiertes Licht in den Lichtleiter eingekoppelt wird, wobei der Lichtleiter derart ausgebildet ist, dass aus dem Lichtleiter wieder austretendes Licht die Oberfläche der Bedieneinheit (2, 6; 18) zumindest teilweise durchleuchtet.

2. Vorrichtung (1; 26) nach Anspruch 1, **dadurch gekennzeichnet, dass** im Lichtleiter und/oder an dessen Oberflächen lichtlenkende Strukturen derart ausgebildet und angeordnet sind, dass eingekoppeltes Licht in Richtung der Oberfläche der Bedieneinheit gelenkt wird.

3. Vorrichtung (1) nach Anspruch 1 oder 2, **dadurch gekennzeichnet, dass** die Bedieneinheit (6) als Handgriff ausgebildet ist.

4. Vorrichtung (1; 26) nach einem der vorstehenden Ansprüche, **dadurch gekennzeichnet, dass** die Bedieneinheit (2, 6; 18) wenigstens eine mit einem Tastsensor (5, 5'; 20) in Wirkverbindung stehende Tastfläche (4, 4'; 19) umfasst, die derart ausgebildet und/oder angeordnet ist, dass die Tastfläche (4, 4'; 19) bei aktivierter Lichtquelle (3, 3'; 21) von dem emittierten Licht durchleuchtet wird.

5. Vorrichtung (1; 26) nach einem der vorstehenden Ansprüche, **dadurch gekennzeichnet, dass** die Bedieneinheit (2, 6; 18) aus transparentem Kunststoff oder aus Glas gefertigt ist.

6. Vorrichtung (1; 26) nach einem der vorstehenden Ansprüche, **dadurch gekennzeichnet, dass** die Bedieneinheit (2, 6; 18) als Touchscreen ausgebildet ist.

7. Vorrichtung (1) nach einem der vorstehenden Ansprüche, **dadurch gekennzeichnet, dass** die Vorrichtung eine Operationsleuchte ist.

8. Vorrichtung (26) nach einem der Ansprüche 1 bis 6, **dadurch gekennzeichnet, dass** die Vorrichtung eine Versorgungseinheit zum Anschließen eines Medizinprodukts ist.

9. Vorrichtung (26) nach einem der Ansprüche 1 bis 6, **dadurch gekennzeichnet, dass** die Vorrichtung ein Medizinprodukt ist.

## Claims

1. A device (1; 26) for use in a medical treatment room, comprising at least one control unit (2, 6; 18), formed as a light guide, and at least one light source (3, 3'; 21), **characterised in that** the light source emits light with a wavelength between 380 nanometres and 420 nanometres, in particular with 405 nanometres, and is arranged in such a way that emitted light is coupled into the light guide, wherein the light guide is formed in such a way that light, emerging from the light guide again, at least partly transilluminates the surface of the control unit (2, 6; 18).

2. A device (1; 26) according to claim 1, **characterised in that** light-directing structures are formed and arranged in the light guide and/or on its surfaces in such a way that coupled-in light is directed in the direction of the surface of the control unit.

3. A device (1) according to claim 1 or 2, **characterised in that** the control unit (6) is formed as a handle.

4. A device (1; 26) according to one of the preceding claims, **characterised in that** the control unit (2, 6; 18) comprises at least one touch surface (4, 4'; 19) that is operatively connected to a touch sensor (5, 5'; 20) and is formed and/or arranged in such a way that the touch surface (4, 4'; 19) is transilluminated by the emitted light when the light source (3, 3'; 21) is activated.

5. A device (1; 26) according to one of the preceding claims, **characterised in that** the control unit (2, 6; 18) is made from transparent plastics material or from glass.

6. A device (1; 26) according to one of the preceding claims, **characterised in that** the control unit (2, 6; 18) is formed as a touchscreen.

7. A device (1) according to one of the preceding claims, **characterised in that** the device is a lighting fixture for an operating room.

8. A device (26) according to one of claims 1 to 6, **characterised in that** the device is a supply unit for the connection of a medical device.

9. A device (26) according to one of claims 1 to 6, **characterised in that** the device is a medical device.

## Revendications

1. Dispositif (1 ; 26) destiné à être utilisé dans un local de traitement médical, comprenant au moins une unité de commande (2, 6 ; 18) conçue ou réalisée sous forme de guide de lumière et au moins une source lumineuse (3, 3' ; 21), **caractérisé en ce que** la source lumineuse émet de la lumière à une longueur d'onde comprise entre 380 nanomètres et 420 nanomètres, et en particulier de 405 nanomètres, et est disposée de telle sorte que la lumière émise est injectée dans le guide de lumière, sachant que le guide de lumière est conçu ou réalisé de telle sorte que la lumière qui ressort à nouveau du guide de lumière éclaire par transmission au moins en partie la surface de l'unité de commande (2, 6 ; 18).

2. Dispositif (1 ; 26) selon la revendication 1, **caractérisé en ce que** des structures dirigeant la lumière dans le guide de lumière et/ou sur ses surfaces sont conçues ou réalisées et disposées de telle sorte que la lumière injectée est dirigée en direction de la surface de l'unité de commande.

3. Dispositif (1) selon la revendication 1 ou 2, **caractérisé en ce que** l'unité de commande (6) est conçue ou réalisée sous forme de poignée.

4. Dispositif (1 ; 26) selon l'une des revendications précédentes, **caractérisé en ce que** l'unité de commande (2, 6 ; 18) comprend au moins une surface tactile (4, 4' ; 19) qui est fonctionnellement reliée à un capteur sensoriel (5, 5' ; 20) et qui est conçue ou réalisée et/ou disposée de telle sorte que la surface tactile (4, 4' ; 19) est éclairée par transmission par la lumière émise lorsque la source lumineuse (3, 3' ; 21) est activée.

5. Dispositif (1 ; 26) selon l'une des revendications précédentes, **caractérisé en ce que** l'unité de commande (2, 6 ; 18) est fabriquée en matière plastique transparente ou en verre.

6. Dispositif (1 ; 26) selon l'une des revendications précédentes, **caractérisé en ce que** l'unité de commande (2, 6 ; 18) est conçue sous forme d'écran tactile.

7. Dispositif (1) selon l'une des revendications précédentes, **caractérisé en ce que** le dispositif est une lampe pour salle d'opération.

8. Dispositif (26) selon l'une des revendications 1 à 6, **caractérisé en ce que** le dispositif est une unité d'alimentation pour le raccordement d'un dispositif médical.

9. Dispositif (26) selon l'une des revendications 1 à 6, **caractérisé en ce que** le dispositif est un dispositif médical.
